# EUROPEAN PATENT APPLICATION

(11) **EP 1 837 026 A1**
(43) Date of publication of application: **26.09.2007**
(21) Application number: 05809590.2
(22) Date of filing: 21.11.2005
(51) Int. Cl.: A61K 31/7048, A23L 1/30, A61P 1/16, C07H 17/07

(54) **HEPATIC FUNCTION REMEDIAL AGENT**

(30) Priority: 24.12.2004 JP 2004373663; 19.04.2005 JP 2005121587
(71) Applicant: KABUSHIKI KAISHA HAYASHIBARA SEIBUTSU KAGAKU KENKYUJO, Okayama-shi, Okayama 700-0907 (JP)
(72) Inventor: MITSUZUMI, Hitoshi, K. K. HAYASHIBARA SEIBUTSU, Okayama-shi, Okayama 700-0907 (JP); YAMADA, Mika, K. K. HAYASHIBARA SEIBUTSU, Okayama-shi, Okayama 700-0907 (JP); MIWA, Yoshikatsu, K. K. HAYASHIBARA SEIBUTSU, Okayama-shi, Okayama 700-0907 (JP); CHAEN, Hiroto, K. K. HAYASHIBARA SEIBUTSU, Okayama-shi, Okayama 700-0907 (JP); MIYAKE, Toshio, K. K. HAYASHIBARA SEIBUTSU, Okayama-shi, Okayama 700-0907 (JP)
(74) Representative: Daniels, Jeffrey Nicholas
(86) International application number: PCT/JP2005/021332
(87) International publication number: WO 2006/067925

(57) **Abstract**

An object of the present invention is to provide a means for improving hepatic function of mammals including humans to normal condition. The present invention achieves the above object by providing a hepatic function-improving agent comprising α-glucosyl-hesperidin as an effective ingredient.

## Description

### TECHNICAL FIELD

The present invention relates to a hepatic function-improving agent, particularly, a hepatic function-improving agent comprising α-glucosyl-hesperidin as an effective ingredient.

### BACKGROUND ART

Liver is one of vital organs that play the essential roles in metabolism. The functions of liver are mainly classified into circulatory function, excretory function, metabolic function, defense-detox function, and hematological function. When any of them is damaged, various characteristic symptoms of hepatopathy, such as feeling of fatigue, feeling of worthlessness, anorexia, jaundice, and low fever, will appear. Prolonged hepatic function disorders will cause lifestyle-related illnesses (adult diseases) such as hepatitis, hepatic cirrhosis, and hepatic carcinoma. It is very important for modern people who spend busy days to keep hepatic functions good for vigorous lives and prevention of lifestyle-related illnesses. However, there have been no ways to keep liver healthy except for some kinds of precepts such as aboiding excessive use of drugs, taking well-balanced meals, and keeping a regular life without overdrink and overwork.

### DISCLOSURE OF INVENTION

Under these circumstances, an object of the present invention is to provide a means to restore hepatic functions in mammals including humans to normal conditions.

The inventors of the present invention have extensively researched on α-glucosyl-hesperidin known as a saccharide derivative of hesperidin with an improved water solubility (for example, see Japanese Patent Kokai No. 346792/99), to meet its use for healthcare and medical field. In consequence, it was found that α-glucosyl-hesperidin has an activity of restoring hepatic functions in mammals including humans, particularly, exhibits marked effect on subjects with elevated serum levels of glutamate oxaloacetate transaminase (hereinafter, abbreviated as "GOT"), glutamate pyruvate transaminase (hereinafter, abbreviated as "GPT") and/or γ-glutamyl transferase (hereinafter, abbreviated as "γ-GT") as hepatic-function indexes and lowers them to normal levels or thereabout, but has little influence upon subjects who are in normal levels of GOT, GPT and γ-GT. The present invention achieves the above object by providing a hepatic function-improving agent comprising α-glucosyl-hesperidin as an effective ingredient and developing its uses.

α-Glucosyl-hesperidin is known to have a marked effect of reducing blood triglyceride (hereinafter, abbreviated as "TG") level when administered to mammals including humans with an elevated TG level (Yamada et al.; Journal of Japanese Society of Nutrition and Food Science, Vol.56, pp.355-363 (2003), Miwa et al.; Journal of Nutritional Science and vitaminology, Vol.51, pp.460-470 (2004)). However, the influences of α-glucosyl-hesperidin upon hepatic functions have never been revealed and the present invention is a novel invention which discloses the use of α-glucosyl-hesperidin as a hepatic function-improving agent.

Administration of the hepatic function-improving agent of the present invention on subjects with elevated serum levels of GOT, GPT, and/or γ-GT out of the normal range reduces them to their normal levels or thereabout and maintains their hepatic functions in good conditions. Additionally, the hepatic function-improving agent of the present invention has little influence upon subjects with normal levels of serum GOT, GPT and γ-GT.

### BRIEF EXPLANATION OF DRAWINGS

FIG. 1 shows the time courses of the serum GOT levels of each group based on the serum GOT level under the administration test of α-glucosyl-hesperidin tablets.
FIG. 2 shows the time courses of the serum GPT levels in each group based on the serum GPT level under the administration test of α-glucosyl-hesperidin tablets.
FIG, 3 shows the time courses of the serum γ-GT levels of each group based on the serum γ-GT level under the administration test of α-glucosyl-hesperidin tablet.
FIG, 4 shows the time courses of the serum TG levels of each group based on the serum TG level under the administration test of α-glucosyl-hesperidin tablets.

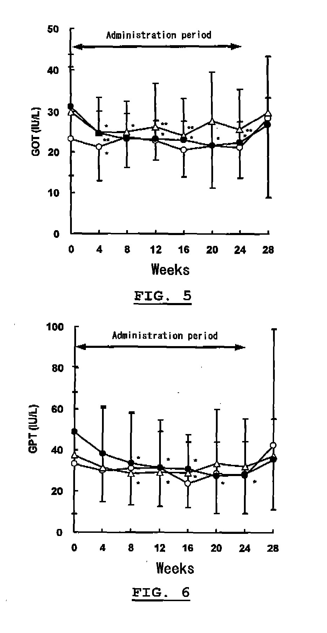

FIG. 5 shows the time courses of the serum GOT levels of each group based on the serum TG level under the administration test of α-glucosyl-hesperidin tablets.

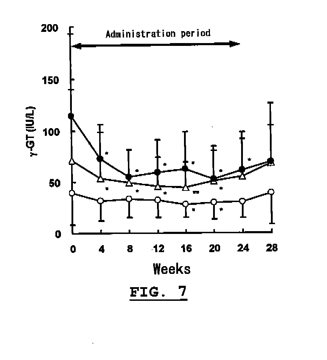

FIG. 6 shows the time courses of the serum GPT levels of each group based on the serum TG level under the administration test of α-glucosyl-hesperidin tablets.
FIG. 7 shows the time courses of the serum γ-GT levels of each group based on the serum TG level under the administration test of α-glucosyl-hesperidin tablets.

### EXPLANATION OF SYMBOLS

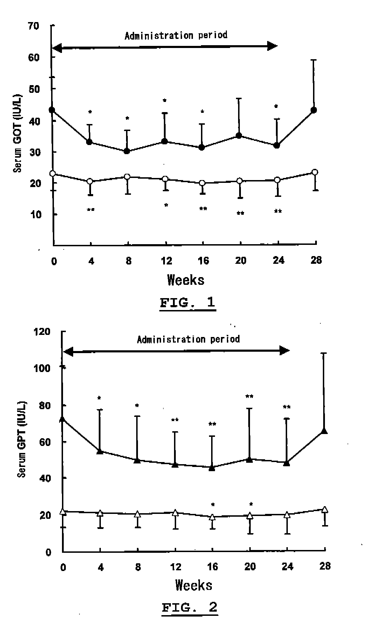

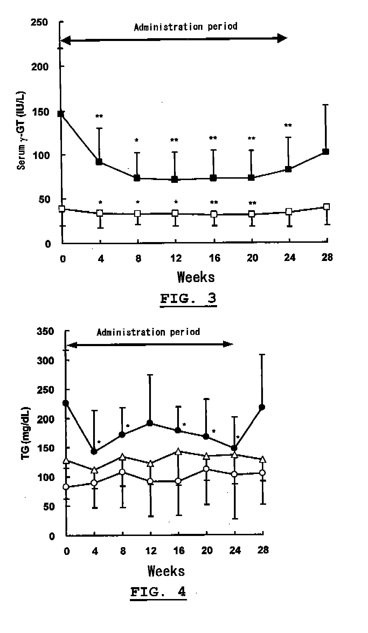

### In FIGs. 1 to 3;

•: Group in "High" levels of the hepatic-function indexes
o: Group in "Normal" levels of the hepatic-function indexes

### In FIGs. 4 to 7;

•: High-TG group
△: Border-TG group
o : Normal-TG group

### Throughout FIGs. 1 to 7;

*: Significantly different at p<0.05 against the data before the administrations;
**: Significantly different at p<0.01 against the data before the administrations

### BEST MODE FOR CARRYING OUT THE INVENTION

The present invention relates to a hepatic function-improving agent comprising α-glucosyl-hesperidin as an effective ingredient. As well known, α-glucosyl-hesperidin is a glucosyl derivative of hesperidin that has the structure in Chemical Formula 1. As long as the hepatic-function improving agent of the present invention contains an effective amount of α-glucosyl-hesperidin as a whole, any preparation method, purity, or form thereof is acceptable.

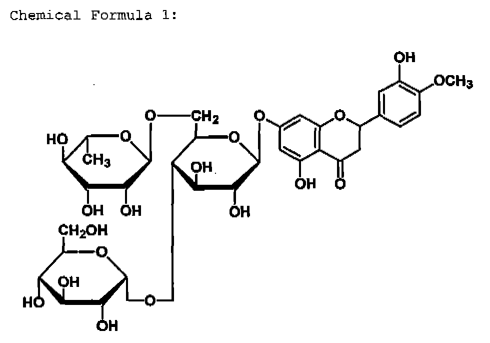

Various preparation methods of α-glucosyl-hesperidin have been known. Biochemical processes using glycosyltransferase are advantegious on a cost front. For example, α-glucosyl-hesperidin is synthesized by allowing saccharide-transfering enzymes such as cyclomaltodextrin glucanotransferase to act on hesperidin in the presence of partially hydrolyzed starch or maltooligosaccharides and allowing a glucoamylase to act on the formed glycosyl-hesperidins. In the present invention, α-glucosyl-hesperidin needs not to be so highly purified, it may be a composition containing unreacted hesperidin or saccharides remaining in a preparation process. Commercially available products in such a form described above, "αG-HESPERIDIN PA®" (a powdery α-glucosyl-hesperidin containing about 68% of α-glucosyl-hesperidin on a dry solid basis, commercialized by Hayashibara Shoji, Inc.) and "αG-HESPERIDIN PS®" (a powdery α-glucosyl-hesperidin containing about 83% of α-glucosyl-hesperidin on a dry solid basis, commercialized by Hayashibara Shoji, Inc.) are advantaegiously used in the present invention.

Administrations of the hepatic function-improving agent of the present invention to mammals whose livers are damaged restore their hepatic functions to their normal conditions. When administered to healthy mammals, it keeps hepatic functions in normal conditions. The particular efficacy of the hepatic function-improving agent of the present invention is demonstrated by reducing the serum levels of GOT, GPT and γ-GT of subjects with elevated levels of GOT, GPT and/or γ-GT into the normal range or thereabout. In the case of humans, the levels of GOT, GPT and γ-GT are reduced to 10 to 35 international units (hereinafter, abbreviated as "IU")/L, 7 to 42 IU/L, and 5 to 83 IU/L, respectively.

The hepatic function-improving agent of the present invention can be α-glucosyl-hesperidin alone or a composition of α-glucosyl-hesperidin and other ingredient(s) that make the intake of α-glucosyl-hesperidin easy. Compositions comprising the hepatic function-improving agent of the present invention are usually provided as foods, beverages or medicines in the form of a liquid, paste, or solid. In the case of foods and beverages, the compositions can be provided as compositions containing general food materials, such as water, alcohols, starch, proteins, fibers, sugars, fats, vitamins, polyphenols, minerals, spices, coloring agents, sweeteners, seasonings, stabilizers, preservatives, and fillers. They may be leveraged for "*tokutei haken-you shokuhin*" (Food for Specified Health Uses), α-Glucosyl-hesperidin can be provided in compositions in the form of a liquid or paste, containing softdrinks such as deep-sea water, carbonated water, vegetable beverages, fruit beverages, tea beverages, milk-based drinks, or peptide beverages, α-Glucosyl-hesperidin can be provided also in compositions in the form of a solid such as powder, granule, capsules and tablets containing powders such as powdery vegetables, powdery fruits, powderytea, powderymilk, and peptide powder. In the case of medicines, α-glucosyl-hesperidin can be provided in compositions containing carriers, fillers, diluents, and/or stabilizers. If necessary, they can comprise one or more bioactive substances such as glycyrrichin, protoporphyrins, tiopronin, malotilate, gultathion, diisopropylamine dichloroacetate, methylmethionine sulfonium chloride, taurine, cyaninodal, interferons, interleukins, vitamins including vitamin B₁, Vitamin B₂, Vitamin B₆ and/or vitamin B₁₂, chinese herbal medicine including *"shosaikoto", "daisaikoto"* and *"saikokeishito"*. Any of the hepatic function-improving agent of the present invention described above generally comprises 0.01 to 100%, favorably 0.1 to 100% by weight of α-glucosyl-hesperidin.

Explaining the use of the hepatic function-improving agent of the present invention with reference to the use for humans, oral intake of the hepatic function-improving agent of the present invention brings out marked effect on the hepatic functions. For example, for the purpose of keeping and enhancing the health or preventing diseases, they can be ingested orally as foods or medicines. The dosage of α-glucosyl-hesperidin is 10 mg to 10 g/shot/adult, preferably 100 mg to 1 g/shot/adult, one to four shots a day or one to seven shots a week.

The following experiments explain the efficacy of the hepatic function-improving agent of the present invention. The test was performed firstly on hamsters as laboratory animals and successively on humans as volunteers.

### Experiment 1

### Administration test of α-glucosyl-hesperidin on hamsters

The animal experiment using hamsters was performed to investigate the influences of the intake of α-glucosyl-hesperidin on hepatic functions.

A group of five golden hamsters of five weeks old was fed on liberal amount of "NMF Lab Animal Diet Powder" (produced by Oriental Yeast Co., Ltd.) with the administration of "αG-HESPERIDIN PS®" (a powdery α-glucosyl-hesperidin containing 82.8% of α-glucosyl-hesperidin, commercialized by Hayashibara Shoji, Inc.) dissolved in distilled water. α-Glucosyl-hesperidin was administered to the hamsters through a stomach sonde at a dose of 0.25 mmole (193 mg)/kg/body weight every day. After two-week administrations of α-glucosyl-hesperidin, the hamsters were fed for additional three weeks in the same way as described above except that their feed was supplemented with 1% by weight of cholesterol. At four hours after the final administrations, the hamsters were anesthetized and underwent laparotomy, and their large-venous blood samples were taken. After the separation of the hamsters' serums by a conventional method, the levels of GOT and GPT were measured. As the control 1, another group was fed and its blood samples were taken in the same way except that distilled water instead of α-glucosyl-hesperidin was administered to the hamsters. As the control 2, a third group was fed and their blood samples were taken in the same way except that hesperidin (a regent grade specimen, commercialized by Wako Pure Chemical Industries, Ltd.) instead of α-glucosyl-hesperidin suspended in distilled water was administered to the hamsters at a dose of 0.25 mmole (153 mg)/kg/body weight. The other group was fed on liberal amount of "NMF Lab Animal Diet Powder" with no administration of the test articles and their blood samples were taken in the same way as describe above, and the serum levels of GOT and GPT were measured to obtain the data before the administrations. These results are shown in Table 1.

**Table 1**

| Groups | Serum GOT (IU/L)* | Serum GPT (IU/L)* |
|---|---|---|
| Before the administrations | 46±8 | 81±18 |
| Distilled water (Control 1) | 171±46 | 418±106 |
| Hesperidin (Control 2) | 101±30 | 298±76 |
| α-Glucosyl-hesperidin | 66±44 | 226±74 |

| | | |
|---|---|---|
| *: Average ± standard deviation (SD) | | |

As evident from the results in Table1, in the group administered I with α-glucosyl-hesperidin or hesperidin (control 2), serum levels of GOT and GPT were kept lower than those in the group administered with distilled water (control 1). The serum levels of GOT and GPT in the group administered with α-glucosyl-hesperidin were lower than those in the group administered with hesperdin, revealing that α-glucosyl-hesperidin has greater effect than that of hesperidin for keeping these hepatic-function indexes at a lower level.

### Experiment 2

### Chronic administration test of α-glucosyl-hesperidin on humans

On the basis of Experiment 1, the influences of chronic intake of α-glucosyl-hesperidin on hepatic functions for humans were investigated with a volunteered test.

Twenty-five male adults (26 to 65 years old) in no medication volunteered as subjects. After four-week preobservation, each subject was administered with an α-glucosyl-hesperidin tablet containing 250 mg of "αG-HESPERIDIN PA®" (a powdery α-glucosyl-hesperidin containing 68.1% of α-glucosyl-hesperidin and 19.5% of hesperidin on a dry solid basis, commercialized by Hayashibara Shoji Inc.), 750 mg of "MALTOSE HHH®" (a maltose product commercialized by Hayashibara Biochemical Laboratories, Inc.) and 30 mg of sucrose stearate ester (commercialized by Mitsubishi-Kagaku Foods Corporation) for 24 weeks and two tablets were administered to each subject once before every bedtime. After completion of the administrations, the subjects were observed for additional four weeks. The blood samples of the subjects were collected and analyzed at four weeks before the initial administrations of the tablets, at the initial administration (0-week administration), at four, eight, 12, 16, 20, and 24-week administrations, and at four weeks after the final administrations. In the early morning on a measurement day bloods were sampled from the subjects who were in over 12-hour fasting from 9 p.m. of the previous day. The subjects spent the test period with usual diet and exercise. Besides, the purpose of the test and the test article were fully explained to the subjects beforehand, and their consents for the test were obtained in writing. The test was performed under sufficient attention according to the spirit of Helsinki Declaration.

The analysis of the blood samples was requested to FALCO biosystems Ltd. and the serum levels of GOT, GPT, and γ-GT as the hepatic-function indexes, and the serum GT level were measured by the conventional method.

### Analytical results 1

### Influences of chronic administration of α-glucosyl-hesperidin on the hepatic functions of human

The results obtained in Experiment 2 were analyzed from the aspect of the hepatic-function indexes, i.e. the serum levels of GOT, GPT, or γ-GT. Based on the level of each index before the test, 25 subjects were categorized into two groups; one with the normal level (Normal), the other with an elevated level (High) out of the normal level. The number of the subjects, the boundary levels of the hepatic-function indexes, and the averages and the standard deviations of the data before the initial administrations in each group were shown in Table 2. The data before the initial administrations were obtained by combining the data at 4 weeks before the initial administrations with the data at the initial administrations (0-week administrations).

**Table 2**

| | Groups based on GOT | | Groups based on GPT | | Groups based on γ-GT | |
|---|---|---|---|---|---|---|
| | Normal (<37 IU/L) | High (37 IU/L≤) | Normal (<42 IU/L) | High (42 IU/L≤) | Normal 1 (<83 IU/L) | High (83 IU/L≤) |
| The number of subjects | 18 | 7 | 16 | 9 | 16 | 9 |
| GOT (IU/L) | 23.1±5.4 | 43.4±10.1 | - | - | - | - |
| GPT (IU/L) | - | - | 21.9±8.6 | 72.6±28.4 | - | - |
| γ-GT (IU/L) | - | - | - | - | 38.6±18.4 | 147.1±73.1 |
| Weight (kg) | 67.3±8.6 | 75.8±11.1 | 67.0±8.9 | 79.4±10.2 | 69.5±11.2 | 70.1±7.6 |
| TG (mg/dL) | 151.6±87.4 | 142.0±38.9 | 129.0±50.8 | 184.3±101.9 | 130.0±55.7 | 182.6±39.6 |

| | | | | | | |
|---|---|---|---|---|---|---|
| Each value except the number of subjects means that of before the initial administration. (Average ± SD} | | | | | | |

The averages and the standard deviations of the serum levels of hepatic-function indexes of the subjects in each group were calculated from the data at four, eight, 12, 16, 20, and 24-week administrations and at four weeks after the final administrations of α-glucosyl-hesperidin tablets. The serum levels of GOT, GPT, and γ-GT in each group were respectively shown in Tables 3, 4, and 5. And, the time courses of these levels from the data before the initial administrations in each group were shown in FIGs. 1, 2, and 3.

**Table 3**

| Groups | Serum GOT (IU/L)* | | | | |
|---|---|---|---|---|---|
| | 4 weeks before the initial administration | 0-week administration | Before the initial administration | 4-week administration | 8-week administration |
| High | 45.1±15.9 | 41.6±5.4 | 43.4±10.1 | 33.1±5.6 | 30.0±6.7 |
| Normal | 22.8±5.1 | 23.4±6.4 | 23.1±5.4 | 20.4±4.4 | 21.9+5.6 |
| Groups | Serum GOT (IU/L)* | | | | |
| | 12-week administration | 16-week administration | 20-week administration | 24-week administration | 4 weeks after the final administration |
| High | 33.0±9.1 | 31.1±7.4 | 34.7±11.5 | 31.6±8.3 | 42.6±15.6 |
| Normal | 21.2±3.8 | 19.7±3.5 | 20.2±5.3 | 20.4±5.1 | 22.8±5.7 |

| | | | | | |
|---|---|---|---|---|---|
| *: Average ± SD **: Average of values of 4 weeks before the initial administration and 0-week administration | | | | | |

**Table 4**

| Groups | Serum GPT (IU/L)* | | | | |
|---|---|---|---|---|---|
| | 4 weeks before the initial administration | 0-week administration | Before the initial administration | 4-week administration | 8-week administration |
| High | 71.2±33.3 | 73.9±26.4 | 72.6±28.4 | 54.8±22.6 | 49.7±23.9 |
| Normal | 22.4±10.2 | 21.4±7.6 | 21.9±8.6 | 21.0±7.9 | 20.3±7.1 |

| Groups | Serum GPT (IU/L)* | | | | |
|---|---|---|---|---|---|
| | 12-week administration | 16-week administration | 20-week administration | 24-week administration | 4 weeks after the final administration |
| High | 47.4±17.8 | 45.7±17.0 | 50.1±27.7 | 48.0±24.1 | 65.8±41.7 |
| Normal | 20.9±8.6 | 18.4±6.2 | 19.2±9.7 | 19.5±10.1 | 22.4±9.0 |

| | | | | | |
|---|---|---|---|---|---|
| *: Average ± SD **: Average of values of 4 weeks before the initial administration and 0-week administration | | | | | |

**Table 5**

| Groups | Serum γ-GT (IU/L)* | | | | |
|---|---|---|---|---|---|
| | 4 weeks before the initial administration | 0-week administration | Before the initial administration | 4-week administration | 8-week administration |
| High | 136.9±72.9 | 157.2±78.5 | 147.1±73.1 | 91.6±38.6 | 73.0±29.3 |
| Normal | 38.3±18.0 | 38.9±20.1 | 38.6±18.4 | 33.8±16.7 | 32.6±12.1 |

| Groups | Serum γ-GT (IU/L)* | | | | |
|---|---|---|---|---|---|
| | 12-week administration | 16-week administration | 20-week administration | 24-week administration | 4 weeks after the final administration |
| High | 71.1±31.1 | 72.2±32.2 | 72.6±31.2 | 81.8±36.2 | 101.1±52.6 |
| Normal | 31.5±11.8 | 31.5±11.8 | 31.3±12.9 | 33.4±15.5 | 38.5±19.4 |

| | | | | | |
|---|---|---|---|---|---|
| * : Average ± SD **: Average of values of 4 weeks before the initial administration and 0-week administration | | | | | |

As evident from the results in Tables 3, 4, and 5, each levels of hepatic-function indexes, i.e. serum GOT, GPT, and γ-GT, decreased within four-week administrations, approached to the normal level or thereabout in eight-week administrations, and remained until 24-week administrations on the subjects each in High-GOT group, High-GPT group, and High-γ-GT group. Each level elevated out of the normal range within four weeks after the final administration, revealing that the effects of restoring the hepatic functions were evidently due to the α-glucosyl-hesperidin tablets. On the other hand, the levels of hepatic-function indexes changed little during the administration test on the subjects each in Normal-GOT group, Normal-GPT group, and Normal-γ-GT group. These results indicate that the α-glucosyl-hesperidin tablet is effective as a hepatic function-improving agent for people in elevated levels of hepatic functions out of the normal range.

### Analytical result 2

### Influences of chronic administration of α-glucosyl-hesperidin on levels of hepatic functions of subjects categorized based on their serum TG level

Another analysis of the results from Experiment 2 was performed on the basis of serum TG levels of the subjects. The subjects were categorized in three groups; "High-TG group" comprising 8 subjects with serum TG levels over 150 mg/dL, "Normal-TG group" comprising 6 subjects with serum TG levels under 110 mg/dL, and "Border-TG group" comprising 11 subjects with serum TG levels from 110 mg/dL to 150 mg/dL. The averages and the standard deviations of the data in each group based on TG level were calculated and analyzed. The serum levels of TG, GOT, GPT, and γ-GT of the subjects in each group obtained from the administration test of α-glucosyl-hesperidin were shown in Tables 6, 7, 8, and 9. The time courses of these levels from the initial administrations were shown in FIGS. 4, 5, 6 and 7.

**Table 6**

| Groups | Serum TG level (mg/dL)* | | | | |
|---|---|---|---|---|---|
| | 4 weeks before the initial administration | 0-week administration | Before the initial administration** | 4-week administration | 8-week administration |
| High-TG | 207.1±89.1 | 245.9±125.1 | 226.5±89.9 | 142.6±70.5 | 172.3±45.5 |
| Border-TG | 135.3±20.6 | 122.3±30.1 | 128.8±13.8 | 111.9±32.8 | 134.4±50.6 |
| Normal-TG | 85.8±25.4 | 79.2±16.6 | 82.5±19.8 | 89.3±42.0 | 106.8±59.1 |

| Groups | Serum TG level (mg/dL)* | | | | |
|---|---|---|---|---|---|
| | 12-week administration | 16-week administration | 20-week administration | 24-week administration | 4 weeks after the final administration |
| High-TG | 190.8±82.7 | 178.1±40.6 | 167.9±64.0 | 147.0±53.8 | 217.3±89.4 |
| Border-TG | 122.2±36.4 | 142.8±58.1 | 134.0±42.0 | 136.4±50.2 | 127.5±35.8 |
| Normal-TG | 91.3±59.2 | 91.0±57.3 | 111.5±60.4 | 101.7±75.3 | 103.7±51.6 |

| | | | | | |
|---|---|---|---|---|---|
| * : Average ± SD **: Average of values of 4 weeks before the initial administration and 0-week administration | | | | | |

**Table 7**

| Groups | Serum GOT (IU/L)* | | | | |
|---|---|---|---|---|---|
| | 4 weeks before the initial administration | 0-week administration | Before the initial administration** | 4-week administration | 8-week administration |
| High-TG | 30.6±11.3 | 31.9±9.2 | 31.3±9.5 | 24.8±5.4 | 23.3±6.2 |
| Border-TG | 30.6±17.3 | 29.3±10.9 | 30.0±13.7 | 24.8±8.5 | 25.0±7.4 |
| Normal-TG | 24.0±8.8 | 22.7±9,5 | 23.3±9.1 | 21.3±8.4 | 23.8±7.7 |

| Groups | Serum GOT (IU/L)* | | | | |
|---|---|---|---|---|---|
| | 12-week administration | 16-week administration | 20-week administration | 24-week administration | 4 weeks after the final administration |
| High-TG | 23.3±4.5 | 23.0±4.6 | 21.6±5.2 | 22.4±5.1 | 26.6±6.5 |
| Border-TG | 26.2±10.6 | 24.1±8.9 | 27.6±11.7 | 25.6±9.6 | 29.6±13.4 |
| Normal-TG | 22.8±9.8 | 20.5±6.7 | 21.7±10.5 | 21.2±7.6 | 28.2±19.3 |

| | | | | | |
|---|---|---|---|---|---|
| * : Average ± SD **: Average of values of 4 weeks before the initial administration and 0-week administration | | | | | |

**Table 8**

| Groups | Serum GPT (IU/L)* | | | | |
|---|---|---|---|---|---|
| | 4 weeks before the initial administration | 0-week administration | Before the initial administration | 4-week administration | 8-week administration |
| High-TG | 47.0±33.6 | 51.1±31.5 | 49.1±31.7 | 38.1±22.6 | 33.5±24.2 |
| Border-TG | 37.9±30.6 | 37.1±27.7 | 37.5±28.5 | 31.4±16.7 | 28.7±15.4 |
| Normal-TG | 34.3±35.4 | 31.8±35.1 | 33.2±35.2 | 29.8±31.4 | 31.2±27.2 |

| Groups | Serum GPT (IU/L)* | | | | |
|---|---|---|---|---|---|
| | 12-week administration | 16-week administration | 20-week administration | 24-week administration | 4 weeks after the final administration |
| High-TG | 31.5±17.8 | 30.6±17.1 | 27.3±16.8 | 28.3±16.0 | 35.5±19.9 |
| Border-TG | 29.1±16.6 | 29.0±16.9 | 33.5±24.3 | 31.9±22.8 | 37.3±26.2 |
| Normal-TG | 31.5±23.2 | 23.7±20.5 | 28.7±31.4 | 27.8±27.6 | 42.3±56.6 |

| | | | | | |
|---|---|---|---|---|---|
| * : Average ± SD **: Average of values of 4 weeks before the initial administration and 0-week administration | | | | | |

**Table 9**

| Groups | Serum γ-GT (IU/L)* | | | | |
|---|---|---|---|---|---|
| | 4 weeks before the initial administration | 0-week administration | Before the initial administration** | 4-week administration | 8-week administration |
| High-TG | 105.9±71.8 | 123.5±89.9 | 114.7±78.6 | 72.9±33.1 | 54.5±26.8 |
| Border-TG | 68.6±68.5 | 74.7±70.3 | 71.7±68.9 | 53.8±44.6 | 49.4±31.9 |
| Normal-TG | 40.3±32.2 | 37.8±28.5 | 39.1±30.3 | 31.7±19.4 | 33.3±17.8 |

| Groups | Serum γ-GT (IU/L)* | | | | |
|---|---|---|---|---|---|
| | 12-week administration | 16-week administration | 20-week administration | 24-week administration | 4 weeks after the final administration |
| High-TG | 58.9±31.8 | 62.3±36.1 | 52.1±27.6 | 61.0±36.5 | 68.8±35.2 |
| Border-TG | 45.8±28.1 | 44.5±24.3 | 50.7±33.5 | 54.6±36.7 | 67.7±57.7 |
| Normal-TG | 31.8±16.7 | 27.7±12.2 | 29.8±16.5 | 30.3±15.9 | 38.5±29.6 |

| | | | | | |
|---|---|---|---|---|---|
| * : Average ± SD **: Average of values of 4 weeks before the initial administration and 0-week administration | | | | | |

As evident form the results in Table 6, the serum TG levels of the subjects in Border-TG group and Normal-TG group little varied by the administrations of the α-glucosyl-hesperidin tablets through the test. On the other hand, the serum TG levels of the subjects in High-TG group dominantly decreased at four-week administrations and came down to 147±53.8 mg/dL, close to the normal level at 24-week administrations. The serum TG level increased to 217±89.4 mg/dL at 4 weeks after the final administrations. These results indicate that the decrease of the serum TG levels were evidently due to the administrations of the α-glucosyl-hesperidin tablets, revealing that α-glucosyl-hesperidin is effective for lowering serum TG level.

As evident from the results in Tables 7, 8, and 9, the serum levels of GPT and γ-GT among the hepatic-function indexes of the subjects in High-TG group decreased to the normal levels within four-week administrations, and remained until 24-week administration. The serum levels of GPT and γ-GT tended to elevate within four weeks after the final administrations, revealing that the effects of restoring the hepatic functions during the administration were evidently due to the α-glucosyl-hesperidin tablets. On this analysis, the serum GOT levels of the subjects in High-TG group were kept in the normal level through the test, effects of the administrations of the α-glucosyl-hesperidin tablets was not exhibited on serum GOT level. On the other hand, the levels of hepatic-function indexes of the subjects in Border-TG group and Normal-TG group remained in the normal levels with little change during the administration test. These analytical results indicate that the α-glucosyl-hesperidin tablets were usable as a hepatic function-improving agent for people with the serum TG levels elevated over 150 mg/dL.

In recent years, fibrate hypolipidamic agents are ocasionally used as medicines which can decrease serum TG level and restore hepatic functions. However, fibrate agents may lower serum TG level under the normal level for patients with the serum TG level in the normal and may lead to ill effects such as hepatic function-injury, gallstone, gastrointestinal injury, so they are contraindicated for patient having hepatic function-injury(for example, "koshikessho-chiryou no shinpo" (Developments in curing of hyperlipemia), nippon rinsho, Vol.59 (supplement 3), pp. 609-617, pp. 618-624 (2001)). Under above situation, a α-glucosyl-hesperidin tablet that has the effects of decreasing serum TG level with little ill effect and restoring hepatic functions can be advantageously usable as hepatic function-improving agent.

The following examples concretely explain embodiments of the present invention.

### Example 1

### Hepatic function-improving agent

Ten parts by weight of L-ascorbic acid, 19 parts by weight of "αG-HESPERIDIN PS®" (a powdery α-glucosyl-hesperidin commercialized by Hayashibara Shoji Inc.), 70 parts by weight of "SUNMALT®" (a maltose product commercialized by Hayashibara Shoji Inc.), and one part by weight of sucrose-stearic acid ester were mixed to homogeneity and made into a tablet comprising α-glucosyl-hesperidin by a conventional tableting method. The product has a readily ingestibility and solubility, and it can be used for supplying vitamin C and used as a hepatic function-improving agent.

### Example 2

### Health food

One hundred fifty parts by weight of "HALLODEX®" (a syrup comprising α-maltosyl trehalose, commercialized by Hayashibara Shoji Inc.) was concentrated to give a moisture content of 15% by weight by heating under a reduced pressure. Then, a gelatin solution prepared by dissolving 13 parts by weight of gelatin into 18 parts by weight of water, two parts by weight of "αG-HESPERIDIN PA®" (a powdery α-glucosyl-hesperidin commercialized by Hayashibara Shoji Inc.), two parts by weight of citric acid, adequate amounts of a coloring and flavor were admixed homogeneously with the above concentrate. The resulting mixture was shaped into a gummy-candy comprising α-glucosyl-hesperidin and packed. The product exhibits satisfactory texture and flavor, and it can be used as a health food for improving or keeping hepatic functions.

### Example 3

### Health food

Three parts by weight of gum base was softened by heating and melting, and then admixed with six parts by weight of "TREHA®" (a hydrous crystalline trehalose commercialized by Hayashibara Shoji Inc.) and one part by weight of "αG-HESPERIDIN PA®" (a powdery α-glucosyl-hesperidin commercialized by Hayashibara Shoji Inc.). The resulting mixture was further admixed with 0.1 part by weight of vitamin E and adequate amounts of a coloring and flavor and kneaded. The resulting mixture was shaped into a chewing gum comprising α-glucosyl-hesperidin and packed. Since the product has satisfactory texture and flavor, it can be used as a health food for improving and keeping hepatic functions.

### Example 4

### Health supplement beverage

Forty parts by weight of isomerized sugar, two parts by weight of "FUNMATSU-MABIT®" (a maltitol product commercialized by Hayashibara Shoji Inc.), 10 parts by weight of rice vinegar, six parts by weight of apple-cider vinegar, two parts by weight of citric acid, two parts by weight of malic acid, two parts by weight of concentrated apple juice, and two parts by weight of "αG-HESPERIDIN PS®" (a powdery α-glucosyl-hesperidin commercialized by Hayashibara Shoji Inc.) were mixed and dissolved to make into a beverage. Since the product has satisfactory texture and flavor, it can be used as a health supplement beverage for improving and keeping hepatic functions.

### Example 5

### Health supplement beverage

Fifty parts by weight of blended green tea leafs were put into 1,500 parts by weight of deionized water at 65°C for five minutes. Successively, the mixture was filtrated through a filter paper to remove tea leafs, and 1, 320 parts by weight of green tea extract was obtained. One part of green tea extract was diluted with four parts of deionized water to reach a concentration suitable for drinking, then admixed with L-ascorbic acid to give a final concentration of 300 ppm, and the pH of the solution was adjusted to pH 6.0 by sodium bicarbonate to make into a preparation. To the preparation, "αG-HESPERIDIN PA®" (α-glucosyl-hesperidin commercialized by Hayashibara Shoji Inc.) and crystalline cycloteterasaccharide penta-hydrate, disclosed in International Patent Application No. WO 02/10361, were added to give respective concentration of 0.2% by weight and 1.5% by weight, on a dry solid basis. Two hundred fifty grams of the resulting preparation was poured into each glass vessel, sealed and sterilized at 121 °C for seven minutes to make into green tea beverage. The product can be used as a health supplement beverage for improving and keeping hepatic functions. Since α-glucosyl-hesperidin and cyclotetrasacaharide exhibit the activity of lowering the levels of cholesterol and triglyceride in blood and other tissues, the product can be advantageously used for preventing or curing lifestyle-related diseases such as hyperlipemia and adipositas with a denotation that it can be used for lowering the amount of lipid.

### Example 6

### Nutritional supplement for tube feeding or oral intake

Thirty parts by weight of "FINETOSE®" (anhydrous crystalline maltose commercialized by Hayashibara Shoji Inc.), three parts by weight of sodium glutamate, two parts by weight of glycine, two parts by weight of sodium chloride, two parts by weight of citric acid, 0.3 part by weight of magnesium carbonate, one part by weight of sodium lactate, four parts by weight of "αG-HESPERIDIN PA®" (a powdery α-glucosyl-hesperidin commercialized by Hayashibara Shoji Inc.), 0.02 part by weight of thiamine, and 0.02 part by weight of riboflavin were mixed and prepared into a composition. Thirty grams each of the resulting composition was put into an aluminum laminate package and heat sealed to make into a nutritional supplement for tube feeding or oral intake. A solution, prepared by dissolving one package of the product into about 250 to 500 ml of sterilized water, can be advantageously used as a nutritional solution for tube feeding or oral intake or an liquid agent for improving or keeping hepatic function.

### INDUSTRTAL APPLICABILITY

As described above, the present invention was made based on a self-finding knowledge that α-glucosyl-hesperidin exhibits a significant hepatic function-improving activity. Habitual use of the hepatic function-improving agent of the present invention effectively keeps or restore health of normal healthy subjects or patients. Particularly, the agent exhibits marked effects for improving the hepatic functions of humans who are in elevated serum levels of GOT, GPT and/or γ-GT out of the normal range. It is also usable as a health food. Further, the hepatic function-improving agent of the present invention can effectively prevents various diseases, caused by hepatic function disorders or diseases accompanied by hepatic function disorders in progress of such diseases, for example, hepatitis, cirrhosis hepatis and hepatoma.

## Claims

1. A hepatic function-improving agent, comprising α-glucosyl hesperidin as an effective ingredient.

2. The agent of claim 1, comprising 0.01 to 100% by weight of α-glucosyl hesperidin.

3. The agent of claim 1, which is in the form of a composition comprising α-glucosyl hesperidin as an effective ingredient and hesperidin, and which comprises at least 0.01% by weight of α-glucosyl hesperidin.

4. The agent of any one of claims 1 to 3, which lowers the serum levels of glutamic-oxaloacetic transaminase (GOT), glutamic-pyruvic transaminase (GPT) and/or γ-glutamyl-transferase (γ-GT) as indexes of hepatic function.

5. A composition in the form of a food or pharmaceutical, comprising the agent of any one of claims 1 to 4.

6. The composition of claim 5, which is a food for specified health uses.

7. The composition of claim 5 or 6, which is in the form of a beverage.

8. The composition of claim 7, wherein said beverage is a soft drink including a tea beverage.
